# EUROPEAN PATENT APPLICATION

(11) **EP 4 830 705 A1**
(43) Date of publication of application: **28.10.2026**
(21) Application number: 24907469.1
(22) Date of filing: 18.12.2024
(51) Int. Cl.: A61K 31/232, A23L 5/00, A23L 33/115, A61K 9/48, A61K 47/36, A61K 47/38, A61K 47/46, A61P 3/00, A61P 3/06, A61P 37/08

(54) **CAPSULE**

(30) Priority: 22.12.2023 JP 2023216842
(71) Applicant: Kao Corporation, Tokyo 103-8210 (JP)
(72) Inventor: OUCHI, Mizue, Tokyo 103-8210 (JP); SAITO, Katsuyoshi, Tokyo 103-8210 (JP)
(74) Representative: Hoffmann Eitle
(86) International application number: PCT/JP2024/044867
(87) International publication number: WO 2025/135085

(57) **Abstract**

Provided is a capsule including a diacylglycerol-containing fat or oil containing ω3 unsaturated fatty acids, in which the discoloration of the fat or oil is suppressed. The capsule includes a capsule film based on a non-animal-derived component, the capsule film being filled with a fat or oil containing 15 mass% or more of diacylglycerols in which 20 mass% or more of constituent fatty acids are ω3 unsaturated fatty acids.

## Description

### Field of the Invention

The present invention relates to a capsule.

### Background of the Invention

It has been reported that ω3 unsaturated fatty acids have various physiological actions, such as a lowering effect on neutral fats and an antiallergic action. The utilization of fats or oils containing a large amount of ω3 unsaturated fatty acids has been desired in recent years. In addition, there is a proposal of dietary supplementation with ω3 unsaturated fatty acids (for example, Patent Document 1).

Meanwhile, it has been reported that a fat or oil containing diacylglycerols at a high concentration has physiological actions, such as suppression of a postprandial increase in blood triglycerides (neutral fats) and a small accumulation amount in the body. Patent Document 2 proposes a soft capsule filled with a fat or oil composition containing: 10.1 wt% to 94.9 wt% of triglycerides; 0.1 wt% to 30 wt% of monoglycerides; and 5 wt% to 59.9 wt% of diglycerides in which 15 wt% to 90 wt% of constituent fatty acids are ω3 unsaturated fatty acids each having less than 20 carbon atoms. Patent Document 3 proposes a soft capsule including a gelatin film filled with a fat or oil containing 15 wt% or more of diglycerides in which the content of an ω3 unsaturated acyl group in a constituent acyl group is 15 wt% or more.
(Patent Document 1) WO 2015/046563 A1
(Patent Document 2) JP-A-2002-138297
(Patent Document 3) JP-A-2007-112806

### Summary of the Invention

The present invention is directed to a capsule, comprising a capsule film based on a non-animal-derived component, the capsule film being filled with a fat or oil containing 15 mass% or more of diacylglycerols in which 20 mass% or more of constituent fatty acids are ω3 unsaturated fatty acids.

### Detailed Description of the Invention

Gelatin is generally used as the capsule film base because of its excellent mechanical strength, film-forming ability, and the like (for example, Patent Documents 1 and 3). It found that, in a gelatin capsule comprising, as a capsule content, a diacylglycerol-containing fat or oil containing ω3 unsaturated fatty acids, a problem with discoloration of the fat or oil over time arose.

Accordingly, the present invention provides a capsule, comprising a diacylglycerol-containing fat or oil containing ω3 unsaturated fatty acids, in which the discoloration of the fat or oil is suppressed.

The present inventors found that a capsule film based on a non-animal-derived component is filled with a diacylglycerol-containing fat or oil containing ω3 unsaturated fatty acids, and consequently the discoloration of the fat or oil is suppressed.

According to the present invention, it provides the capsule, comprising, as a capsule content, a diacylglycerol-containing fat or oil containing ω3 unsaturated fatty acids, while exhibiting the suppressed discoloration of the fat or oil and the excellent oxidative stability.

### [Capsule]

The capsule of the present invention contains a capsule film based on a non-animal-derived component, the capsule film being filled with a fat or oil containing 15 mass% or more of diacylglycerols in which 20 mass% or more of constituent fatty acids are ω3 unsaturated fatty acids. In the present invention, the fat or oil contains any one or more of monoacylglycerols, diacylglycerols, and triacylglycerols.

The term "fat or oil containing 15 mass% or more of diacylglycerols in which 20 mass% or more of constituent fatty acids are ω3 unsaturated fatty acids" as used herein is hereinafter sometimes simply referred to as "fat or oil of the present invention."

In the fat or oil of the present invention, the content of the ω3 unsaturated fatty acids in the constituent fatty acids of the diacylglycerols is 20 mass% (hereinafter simply referred to as "%") or more. The content of the ω3 unsaturated fatty acids in the constituent fatty acids of the diacylglycerols is preferably 25% or more, more preferably 30% or more, even more preferably 40% or more, even more preferably 45% or more, even more preferably 50% or more, even more preferably 52% or more, from the standpoint of physiological effects, and is preferably 99% or less, more preferably 98% or less, even more preferably 95% or less, even more preferably 85% or less, even more preferably 80% or less, even more preferably 75% or less, even more preferably 70% or less, even more preferably 65% or less, even more preferably 60% or less, even more preferably 58% or less, from the standpoint of oxidative stability.

The content of the ω3 unsaturated fatty acids in the constituent fatty acids of the diacylglycerols is 20% or more, preferably from 20% to 99%, more preferably from 25% to 98%, even more preferably from 30% to 95%, even more preferably from 40% to 85%, even more preferably from 45% to 80%, even more preferably from 50% to 75%, even more preferably from 50% to 70%, even more preferably from 50% to 65%, even more preferably from 50% to 60%, even more preferably from 50% to 58%, even more preferably from 52% to 58%.

The amount of a fatty acid as used herein refers to an amount obtained by converting the fatty acid to a free fatty acid.

Examples of the ω3 unsaturated fatty acid may include α-linolenic acid (C18:3, ALA), eicosapentaenoic acid (C20:5, EPA), docosapentaenoic acid (C22:5), and docosahexaenoic acid (C22:6, DHA). Of those, at least one selected from the group consisting of α-linolenic acid (C18:3, ALA), eicosapentaenoic acid (C20:5, EPA), and docosahexaenoic acid (C22:6, DHA) is preferred from the standpoint of physiological activity and the standpoint of ease of obtaining the effect of the present invention. Furthermore, α-linolenic acid (C18:3, ALA) is even more preferred from the standpoint of oxidative stability.

The constituent fatty acids of the diacylglycerols other than the ω3 unsaturated fatty acids are not particularly limited, and may be saturated fatty acids or unsaturated fatty acids.

From the standpoints of the taste and flavor, and industrial productivity of the fat or oil, the content of the unsaturated fatty acids in the constituent fatty acids of the diacylglycerols is preferably from 60% to 100%, more preferably from 70% to 99.8%, even more preferably from 80% to 99.5%. The number of carbon atoms of the unsaturated fatty acid is preferably from 14 to 24, more preferably from 16 to 22, from the standpoint of physiological effects.

In particular, the content of linoleic acid (C18:2) in the constituent fatty acids of the diacylglycerols is preferably 0.4% or more, more preferably 0.5% or more, even more preferably 1% or more, even more preferably 1.5% or more, and is preferably 30% or less, more preferably 20% or less, even more preferably 10% or less, from the standpoint of the industrial productivity of the fat or oil.

In addition, the content of oleic acid (C18:1) in the constituent fatty acids of the diacylglycerols is preferably 1% or more, more preferably 5% or more, even more preferably 7% or more, even more preferably 10% or more, and is preferably 30% or less, more preferably 25% or less, even more preferably 20% or less, from the standpoint of the industrial productivity of the fat or oil.

The total content of the saturated fatty acids in the constituent fatty acids of the diacylglycerols is preferably 25% or less, more preferably 20% or less, even more preferably 15% or less, even more preferably 12% or less, even more preferably 10% or less, and is preferably 0.5% or more, from the standpoints of appearance, physiological effects, and the industrial productivity of the fat or oil.

The total content of the saturated fatty acids in the constituent fatty acids of the diacylglycerols is preferably from 0% to 25%, more preferably from 0% to 20%, more preferably from 0% to 15%, even more preferably from 0.5% to 12%, even more preferably from 0.5% to 10%.

Herein, the number of carbon atoms of the saturated fatty acid is preferably from 14 to 24, more preferably from 16 to 22, even more preferably 16, 18, or 20.

In the fat or oil of the present invention, the content of the diacylglycerols is 15% or more. The content of the diacylglycerols in the fat or oil of the present invention is preferably 20% or more, more preferably 25% or more, even more preferably 30% or more, even more preferably 50% or more, even more preferably 60% or more, even more preferably 70% or more, even more preferably 80% or more, from the standpoint of effectively expressing effects and the standpoint of physiological effects, and is preferably 99.5% or less, more preferably 98% or less, even more preferably 97% or less, even more preferably 90% or less, from the standpoint of industrial productivity.

The content of the diacylglycerols in the fat or oil of the present invention is 15% or more, preferably from 15% to 99.5%, more preferably from 20% to 98%, even more preferably from 25% to 98%, even more preferably from 30% to 98%, even more preferably from 50% to 97%, even more preferably from 60% to 97%, even more preferably from 70% to 90%, even more preferably from 80% to 90%.

The fat or oil of the present invention may contain triacylglycerols, and the content thereof is preferably 1% or more, more preferably 2% or more, even more preferably 5% or more, even more preferably 10% or more, and is preferably 85% or less, more preferably 80% or less, even more preferably 75% or less, even more preferably 50% or less, even more preferably 25% or less, from the standpoint of the industrial productivity of the fat or oil.

The content of monoacylglycerols in the fat or oil of the present invention is preferably 5% or less, more preferably 3% or less, even more preferably 2% or less, even more preferably 1.5% or less, and is preferably more than 0%, from the standpoints of taste and flavor, the industrial productivity of the fat or oil, and oxidative stability. The content of the monoacylglycerols in the fat or oil may be 0%.

The fat or oil of the present invention may contain a free fatty acid or a salt thereof as an impurity. The content of the free fatty acid or the salt thereof in the fat or oil is preferably 3% or less, more preferably 2% or less, even more preferably 1% or less, and is preferably more than 0%, from the standpoints of taste and flavor, and oxidative stability. The content of the free fatty acid or the salt thereof in the fat or oil may be 0%.

In the fat or oil of the present invention, the constituent fatty acids of the fat or oil may be identical in composition to, or different therein from, the above-mentioned constituent fatty acids of the diacylglycerols, but are preferably identical.

In the present invention, the content of the ω3 unsaturated fatty acids in the constituent fatty acids of the fat or oil of the present invention is preferably from 20% to 80%, more preferably from 25% to 70%, even more preferably from 30% to 70%, even more preferably from 40% to 70%, even more preferably from 45% to 70%, even more preferably from 50% to 65%, even more preferably from 52% to 60%, from the standpoint of effectively expressing effects and the standpoint of physiological effects.

In addition, the content of the unsaturated fatty acids in the constituent fatty acids of the fat or oil of the present invention is preferably from 60% to 100%, more preferably from 70% to 99.8%, even more preferably from 80% to 99.5%.

The fat or oil of the present invention may be obtained in accordance with a conventional method, for example, by: an esterification reaction between fatty acids derived from a fat or oil, and glycerin; or a transesterification reaction (glycerolysis) between a fat or oil, and glycerin. A general edible fat or oil may be mixed as required.

The esterification reaction and the glycerolysis reaction are broadly classified into: a chemical method involving using a chemical catalyst, such as an alkali metal or an alloy thereof, an oxide or hydroxide of an alkali metal or an alkaline earth metal, or an alkoxide of an alkali metal or an alkaline earth metal; and an enzymatic method involving using an enzyme such as a lipase.

In the present invention, the fat or oil (edible fat or oil) may be any one of a plant-derived fat or oil or an animal-derived fat or oil. Examples thereof may include: plant-derived fats or oils, such as soybean oil, rapeseed oil, safflower oil, rice oil, corn oil, sunflower oil, cotton seed oil, olive oil, sesame oil, peanut oil, Job's tears seed oil, wheat germ oil, Japanese basil oil, linseed oil, perilla oil, chia seed oil, sacha inchi oil, walnut oil, kiwi seed oil, salvia seed oil, grape seed oil, macadamia nut oil, hazelnut oil, pumpkin seed oil, camellia oil, tea seed oil, borage oil, palm oil, palm olein, palm stearin, coconut oil, palm kernel oil, cacao butter, sal butter, shea butter, and algae oil; animal-derived fats or oils, such as fish oil, seal oil, whale oil, lard, beef tallow, and butter fat; and microbial oils including a fat or oil derived from a polyunsaturated fatty acid-producing microorganism such as Zygomycetes; and transesterified fats or oils, hydrogenated oils, and fractionated oils thereof. The fats or oils may be used alone or in combination thereof.

Of those, from the standpoint of usability, a liquid fat or oil excellent in low-temperature resistance is preferably used, and one or more of fats or oils selected from the group consisting of fats or oils rich in ω3 unsaturated fatty acids, such as Japanese basil oil, linseed oil, perilla oil, chia seed oil, sacha inchi oil, algae oil, fish oil, seal oil, whale oil, and a fat or oil derived from a polyunsaturated fatty acid-producing microorganism are more preferably used. The liquid fat or oil means a fat or oil that is in a liquid state at 20°C, when determined in accordance with a cold test described in Standard Methods for Analysis of Fats, Oils and Related Materials 2.3.8-27.

The fatty acids derived from a fat or oil may be obtained by hydrolyzing a fat or oil. As a method of hydrolyzing the fat or oil, there are given a high-temperature and high-pressure degradation method and an enzymatic degradation method. The high-temperature and high-pressure degradation method is a method involving adding water to a fat or oil, and subjecting the mixture to a reaction under high-temperature and high-pressure conditions, to thereby provide fatty acids and glycerin. In addition, the enzymatic degradation method is a method involving adding water to a fat or oil, and subjecting the mixture to a reaction under a low-temperature condition using a fat or oil hydrolytic enzyme as a catalyst, to thereby provide fatty acids and glycerin.

The hydrolysis reaction may be performed in accordance with a conventional method.

After the hydrolysis of the fat or oil, it is preferred that a hydrolysate be fractionated and a solid be removed. As a fractionation method, there are given a solvent fractionation method, a spontaneous fractionation method (dry fractionation method), and a wetting agent fractionation method.

As means for removing a precipitated solid, there are given, for example, settled separation, filtration, centrifugation, and a method involving mixing fatty acids with an aqueous solution of a wetting agent, followed by separation.

The esterification reaction between the fatty acids derived from a fat or oil, and glycerin is preferably performed by an enzymatic method under mild conditions, which is excellent from the standpoint of, for example, taste and flavor.

The usage amount of the enzyme may be appropriately determined in consideration of the activity of the enzyme. From the standpoint of increasing a reaction rate, when an immobilized enzyme is used, the usage amount is preferably from 1% to 30%, more preferably from 2% to 20%, with respect to the total mass of raw materials for the esterification reaction.

The reaction temperature of the esterification reaction is preferably from 0°C to 100°C, more preferably from 20°C to 80°C, even more preferably from 30°C to 60°C, from the standpoint of increasing a reaction rate and the standpoint of suppressing inactivation of an enzyme. In addition, the reaction time is preferably 15 hours or less, more preferably from 1 hour to 12 hours, even more preferably from 2 hours to 10 hours, from the standpoint of industrial productivity.

As means for bringing the fatty acids and glycerin into contact with each other, there are given, for example, immersion, stirring, and a method involving passing a liquid through a column packed with an immobilized lipase through use of a pump or the like.

The transesterification reaction (glycerolysis) between the fat or oil and glycerin is preferably performed by a chemical method from the standpoint of reactivity.

The usage amount of the catalyst is preferably from 0.001% to 3%, more preferably from 0.005% to 2%, even more preferably from 0.01% to 1%, with respect to the mass of raw materials for the reaction, from the standpoint of reactivity.

In the present invention, the pressure in the reaction system during the transesterification reaction is not particularly specified, and the reaction may be performed under normal pressure or reduced pressure. In the case of normal pressure, it is preferred that the reaction be performed under a nitrogen stream from the standpoint of reactivity.

After the esterification reaction or the transesterification reaction (glycerolysis), a refinement process to be generally used for a fat or oil may be performed. Specific examples thereof may include processes, such as distillation treatment, acid treatment, water washing, decoloration, and deodorization.

It is preferred that an antioxidant be added to the fat or oil of the present invention from the standpoints of, for example, taste and flavor, oxidative stability, and suppression of discoloration. The addition amount of the antioxidant is preferably from 0.005% to 3%, more preferably from 0.04% to 2%, even more preferably from 0.08% to 1%, even more preferably from 0.2% to 0.8%, with respect to the mass of the fat or oil.

The antioxidant is not particularly limited as long as the antioxidant is used for food, but is preferably at least one selected from the group consisting of, for example, a natural antioxidant, lecithin, tocopherol, ascorbyl palmitate, ascorbyl stearate, dibutylhydroxytoluene (BHT), and butylated hydroxyanisole (BHA).

In the present invention, the content of the fat or oil of the present invention in the capsule contents may be appropriately selected, but for example, is preferably 1% or more, more preferably 2% or more, even more preferably 5% or more, and is preferably 100%, and is preferably 99% or less when any other component is incorporated, from the standpoint of physiological effects and the standpoint of easily ingesting an effective amount.

The capsule contents may contain any other component, such as a fat or oil other than the fat or oil of the present invention, a sweetener, an acidulant, an amino acid, a protein, a vitamin, a mineral, a plant extract, a colorant, an emulsifier, a defoaming agent, a preservative, and a flavoring, as desired. The content of each of those components may be appropriately set within the range that does not impair the object of the present invention.

The capsule of the present invention has a capsule film based on a non-animal-derived component. In the case where the non-animal-derived component is used as the film base instead of animal-derived gelatin, even when the fat or oil of the present invention is incorporated as the capsule content, the discoloration of the fat or oil can be suppressed. In addition, the capsule has such effects that the oxidative stability of the fat or oil is high, and the capsule has less odor derived from the capsule content as compared to a gelatin capsule.

Examples of the non-animal-derived component may include a plant-derived component, a seaweed-derived component, and a microorganism-derived component. The non-animal-derived component is preferably a plant-derived polysaccharide, a seaweed-derived polysaccharide, or a microorganism-derived polysaccharide. Those components may be used alone or in combination thereof.

Examples of the plant-derived component may include starch, modified starch (e.g., acid-treated starch, phosphate-crosslinked starch, wet-heat-treated starch, phosphate-crosslinked esterified starch, phosphate-crosslinked hydroxypropylated starch, acetylated starch, phosphorylated starch, starch octenyl succinate, hydroxypropylated starch, acetylated phosphate-crosslinked starch), dextrin, maltodextrin, indigestible dextrin, locust bean gum, guar gum, tamarind gum, gum arabic, karaya gum, pectin, inulin, a cellulose derivative (e.g., methylcellulose (MC), ethylcellulose (EC), hydroxypropylcellulose (HPC), low-substituted hydroxypropylcellulose (L-HPC), hydroxypropylmethylcellulose (HPMC), or carboxymethylcellulose (CMC)), glucomannan, galactan, and soybean polysaccharide.

Examples of the seaweed-derived component may include potassium alginate, sodium alginate, an alginate ester, carrageenan (λ-carrageenan, ι-carrageenan, or κ-carrageenan), and agar.

Examples of the microorganism-derived component may include xanthan gum, gellan gum, and pullulan.

Of those, from the standpoint of suppressing the discoloration of the fat or oil of the present invention, at least one selected from the group consisting of starch, modified starch, dextrin, gum arabic, pectin, a cellulose derivative, glucomannan, carrageenan, gellan gum, xanthan gum, locust bean gum, guar gum, potassium alginate, sodium alginate, an alginate ester, and pullulan is preferably used, and at least one selected from the group consisting of starch, modified starch, dextrin, carrageenan, gellan gum, xanthan gum, locust bean gum, guar gum, and pullulan is more preferably used.

The capsule of the present invention may be a hard capsule or a soft capsule. A soft capsule is preferred from the standpoints of sealability and oxidative stability.

The shape of the capsule is not particularly limited, and examples thereof may include a spherical shape, an elliptical shape, an oblong shape, a tube shape, and a suppository shape.

The content of the non-animal-derived component in the capsule film of the present invention is preferably 60% or more, and is preferably 92.5% or less.

When the capsule of the present invention is a hard capsule, the content of the non-animal-derived component in the capsule film is preferably 86% or more, and is preferably 92.5% or less.

When the capsule of the present invention is a soft capsule, the content of the non-animal-derived component in the capsule film is preferably 60% or more, more preferably 62% or more, even more preferably 63% or more, still even more preferably 65% or more, and is preferably 85% or less, more preferably 80% or less, even more preferably 78% or less, still even more preferably 75% or less.

The capsule film may further contain various additives to be used for a capsule film as required, such as a plasticizer (e.g., glycerin, sorbitol, erythritol, propylene glycol, or polyethylene glycol), a colorant, a sweetener, an antiseptic, an anti-sticking agent, a water activity lowering agent, a pH adjuster, a flavoring, and water. The content of each of those components may be appropriately set within the range that does not impair the object of the present invention. The content of the plasticizer in the capsule film is, for example, from 5% to 70%, preferably from 10% to 60%.

In the capsule of the present invention, the mass ratio of the capsule film to the capsule contents, [(capsule film)/(capsule contents)], may be appropriately selected, but is preferably from 0.01 to 99, more preferably from 0.01 to 90, more preferably from 0.01 to 80, more preferably from 0.02 to 60, more preferably from 0.02 to 40, more preferably from 0.02 to 20, more preferably from 0.02 to 10, more preferably from 0.05 to 10, more preferably from 0.05 to 5, even more preferably from 0.1 to 5, from the standpoint of easily ingesting an effective amount and the standpoint of industrial productivity.

In the capsule of the present invention, the ratio of the content of the diacylglycerols in the fat or oil to the content of the non-animal-derived component in the capsule film, [(DAG)/(non-animal-derived component)], is preferably 0.309 or more, and is preferably 1.47 or less.

When the capsule of the present invention is a hard capsule, the ratio of the content of the diacylglycerols in the fat or oil to the content of the non-animal-derived component in the capsule film, [(DAG)/(non-animal-derived component)], is preferably 0.309 or more, more preferably 0.588 or more, and is preferably 0.988 or less, more preferably 0.919 or less, even more preferably 0.876 or less.

When the capsule of the present invention is a soft capsule, the ratioof the content of the diacylglycerols in the fat or oil to the content of the non-animal-derived component in the capsule film, [(DAG)/(non-animal-derived component)], is preferably 0.410 or more, more preferably 0.778 or more, and is preferably 1.47 or less, more preferably 1.31 or less.

In the capsule of the present invention, the ratio of the content of the ω3 unsaturated fatty acids in the constituent fatty acids of the diacylglycerols to the content of the non-animal-derived component in the capsule film, [(ω3)/(non-animal-derived component)], is preferably 0.565 or more, and is preferably 1.05 or less.

When the capsule of the present invention is a hard capsule, the ratioof the content of the ω3 unsaturated fatty acids in the constituent fatty acids of the diacylglycerols to the content of the non-animal-derived component in the capsule film, [(ω3)/(non-animal-derived component)], is preferably 0.565 or more, more preferably 0.593 or more, and is preferably 0.651 or less, more preferably 0.608 or less, even more preferably 0.602 or less.

When the capsule of the present invention is a soft capsule, the ratio of the content of the ω3 unsaturated fatty acids in the constituent fatty acids of the diacylglycerols to the content of the non-animal-derived component in the capsule film, [(ω3)/(non-animal-derived component)], is preferably 0.784 or more, more preferably 0.797 or more, and is preferably 1.05 or less, more preferably 0.805 or less.

In the capsule of the present invention, the ratio of the content of triacylglycerols in the fat or oil to the content of the non-animal-derived component in the capsule, [(TAG)/(non-animal-derived component)], film is preferably 0.0640 or more, and is preferably 1.12 or less.

When the capsule of the present invention is a hard capsule, the ratio of the content of triacylglycerols in the fat or oil to the content of the non-animal-derived component in the capsule film, [(TAG)/(non-animal-derived component)], is preferably 0.144 or more, more preferably 0.155 or more, even more preferably 0.269 or more, and is preferably 0.845 or less, more preferably 0.567 or less.

When the capsule of the present invention is a soft capsule, the ratio of the content of triacylglycerols in the fat or oil to the content of the non-animal-derived component in the capsule film, [(TAG)/(non-animal-derived component)], is preferably 0.0640 or more, more preferably 0.205 or more, and is preferably 1.12 or less, more preferably 0.751 or less.

The capsule of the present invention may be produced in accordance with a conventional method. For example, a hard capsule may be produced by preparing a capsule film based on a non-animal-derived component, filling the capsule film with the fat or oil of the present invention after drying, and sealing the capsule. In addition, a soft capsule may be produced by filling a capsule film based on a non-animal-derived component with the fat or oil of the present invention, followed by molding and drying, through use of, for example, a rotary-type (e.g., rotary die-type), seamless-type, or flat plate-type production method.

The capsule of the present invention may be used for various products, such as a pharmaceutical, a quasi-drug, a cosmetic preparation, and a food. Examples of the food may include foods for specified health use and foods with function claims claiming to have the physiological effects of ω3 unsaturated fatty acids and diacylglycerols as well as general foods.

### [Second Aspect of Capsule]

A capsule according to a second aspect of the present invention contains a capsule film including a non-animal-derived component, the capsule film being filled with a fat or oil containing 15% or more of diacylglycerols in which 20% or more of constituent fatty acids are ω3 unsaturated fatty acids.

In the capsule according to the second aspect of the present invention, the content of an animal-derived protein in components for forming the capsule film other than water is preferably less than 5%, more preferably less than 3%, even more preferably less than 1%, still even more preferably less than 0.1%, and the capsule is still even more preferably substantially free of the anima-derived protein. The phrase "substantially free" as used herein is a concept encompassing not only the fact that animal-derived protein is completely absent in the capsule film, but also the fact that its concentration is less than the detection limit.

The animal-derived protein is preferably, for example, collagen.

The specific configurations of the fat or oil, the capsule contents, the non-animal-derived component, the additive, the mass ratio of the capsule film to the capsule contents, the ratio of the capsule film component to the fat or oil component, the method of producing the capsule, and the like may be the same as those described above.

### [Method of Suppressing Discoloration of Fat or Oil]

A method of suppressing the discoloration of the fat or oil of the present invention may be achieved by using a capsule film based on a non-animal-derived component or a capsule film including a non-animal-derived component, as the capsule film to be filled with a fat or oil containing 15% or more of diacylglycerols in which 20% or more of constituent fatty acids are ω3 unsaturated fatty acids.

The specific configurations of the fat or oil, the capsule contents, the non-animal-derived component, the animal-derived component, the additive, the mass ratio of the capsule film to the capsule contents, the ratio of the capsule film component to the fat or oil component, the method of producing the capsule, and the like may be the same as those described above.

In relation to the above-mentioned embodiments, the present invention further discloses the following aspects.
<1> A capsule, comprising a capsule film based on a non-animal-derived component, the capsule film being filled with a fat or oil containing 15 mass% or more of diacylglycerols in which 20 mass% or more of constituent fatty acids are ω3 unsaturated fatty acids.
<2> The capsule according to <1>, wherein the content of the ω3 unsaturated fatty acids in the constituent fatty acids of the diacylglycerols is preferably 25 mass% or more, even more preferably 30 mass% or more, even more preferably 40 mass% or more, even more preferably 45 mass% or more, even more preferably 50 mass% or more, even more preferably 52 mass% or more, and is preferably 99 mass% or less, more preferably 98 mass% or less, even more preferably 95 mass% or less, even more preferably 85 mass% or less, even more preferably 80 mass% or less, even more preferably 75 mass% or less, even more preferably 70 mass% or less, even more preferably 65 mass% or less, even more preferably 60 mass% or less, even more preferably 58 mass% or less.
<3> The capsule according to <1>, wherein the content of the ω3 unsaturated fatty acids in the constituent fatty acids of the diacylglycerols is preferably from 20 mass% to 99 mass%, more preferably from 25 mass% to 98 mass%, even more preferably from 30 mass% to 95 mass%, even more preferably from 40 mass% to 85 mass%, even more preferably from 45 mass% to 80 mass%, even more preferably from 50 mass% to 75 mass%, even more preferably from 50 mass% to 70 mass%, even more preferably from 50 mass% to 65 mass%, even more preferably from 50 mass% to 60 mass%, even more preferably from 50 mass% to 58 mass%, even more preferably from 52 mass% to 58 mass%.
<4> The capsule according to any one of <1> to <3>, wherein the ω3 unsaturated fatty acid is at least one selected from the group consisting of α-linolenic acid, eicosapentaenoic acid, and docosahexaenoic acid.
<5> The capsule according to any one of <1> to <4>, wherein the content of linoleic acid (C18:2) in the constituent fatty acids of the diacylglycerols is preferably 0.4 mass% or more, more preferably 0.5 mass% or more, even more preferably 1 mass% or more, even more preferably 1.5 mass% or more, and is preferably 30 mass% or less, more preferably 20 mass% or less, even more preferably 10 mass% or less.
<6> The capsule according to any one of <1> to <5>, wherein the content of oleic acid (C18:1) in the constituent fatty acids of the diacylglycerols is preferably 1 mass% or more, more preferably 5 mass% or more, even more preferably 7 mass% or more, even more preferably 10 mass% or more, and is preferably 30 mass% or less, more preferably 25 mass% or less, even more preferably 20 mass% or less.
<7> The capsule according to any one of <1> to <6>, wherein the content of the diacylglycerols in the fat or oil is preferably 20 mass% or more, more preferably 25 mass% or more, even more preferably 30 mass% or more, even more preferably 50 mass% or more, even more preferably 60 mass% or more, even more preferably 70 mass% or more, even more preferably 80 mass% or more, and is preferably 99.5 mass% or less, more preferably 98 mass% or less, even more preferably 97 mass% or less, even more preferably 90 mass% or less.
<8> The capsule according to any one of <1> to <6>, wherein the content of the diacylglycerols in the fat or oil is preferably from 15 mass% to 99.5 mass%, more preferably from 20 mass% to 98 mass%, even more preferably from 25 mass% to 98 mass%, even more preferably from 30 mass% to 98 mass%, even more preferably from 50 mass% to 97 mass%, even more preferably from 60 mass% to 97 mass%, even more preferably from 70 mass% to 90 mass%, even more preferably from 80 mass% to 90 mass%.
<9> The capsule according to any one of <1> to <8>, wherein the content of triacylglycerols in the fat or oil is preferably 1 mass% or more, more preferably 2 mass% or more, even more preferably 5 mass% or more, even more preferably 10 mass% or more, and is preferably 85 mass% or less, more preferably 80 mass% or less, even more preferably 75 mass% or less, even more preferably 50 mass% or less, even more preferably 25 mass% or less.
<10> The capsule according to any one of <1> to <9>, wherein the content of monoacylglycerols in the fat or oil is preferably 5 mass% or less, more preferably 3 mass% or less, even more preferably 2 mass% or less, even more preferably 1.5 mass% or less, and is preferably more than 0 mass%.
<11> The capsule according to any one of <1> to <10>, wherein the content of a free fatty acid or a salt thereof in the fat or oil is preferably 3 mass% or less, more preferably 2 mass% or less, even more preferably 1 mass% or less, and is preferably more than 0 mass%.
<12> The capsule according to any one of <1> to <11>, wherein the content of the ω3 unsaturated fatty acids in the constituent fatty acids of the fat or oil is preferably from 20 mass% to 80 mass%, more preferably from 25 mass% to 70 mass%, even more preferably from 30 mass% to 70 mass%, even more preferably from 40 mass% to 70 mass%, even more preferably from 45 mass% to 70 mass%, even more preferably from 50 mass% to 65 mass%, even more preferably from 52 mass% to 60 mass%.
<13> The capsule according to any one of <1> to <12>, wherein the fat or oil comprises a fat or oil obtained by an esterification reaction between a fatty acid derived from one or more of fats or oils selected from the group consisting of Japanese basil oil, linseed oil, perilla oil, chia seed oil, sacha inchi oil, and algae oil, and glycerin, or a transesterification reaction (glycerolysis) between one or more of fats or oils selected from the group consisting of Japanese basil oil, linseed oil, perilla oil, chia seed oil, sacha inchi oil, and algae oil, and glycerin.
<14> The capsule according to any one of <1> to <13>, wherein the fat or oil further comprises an antioxidant.
<15> The capsule according to <14>, wherein the addition amount of the antioxidant is preferably from 0.005 mass% to 3 mass%, more preferably from 0.04 mass% to 2 mass%, even more preferably from 0.08 mass% to 1 mass%, even more preferably from 0.2 mass% to 0.8 mass%, with respect to the mass of the fat or oil.
<16> The capsule according to <14> or <15>, wherein the antioxidant is at least one selected from the group consisting of, for example, a natural antioxidant, lecithin, tocopherol, ascorbyl palmitate, ascorbyl stearate, dibutylhydroxytoluene (BHT), and butylated hydroxyanisole (BHA).
<17> The capsule according to any one of <1> to <16>, wherein the non-animal-derived component comprises at least one selected from the group consisting of a plant-derived component, a seaweed-derived component, and a microorganism-derived component.
<18> The capsule according to any one of <1> to <16>, wherein the non-animal-derived component comprises at least one selected from the group consisting of polysaccharides of a plant-derived component, a seaweed-derived component, and a microorganism-derived component.
<19> The capsule according to any one of <1> to <16>, wherein the non-animal-derived component comprises at least one selected from the group consisting of starch, modified starch, dextrin, gum arabic, pectin, a cellulose derivative, glucomannan, carrageenan, gellan gum, xanthan gum, locust bean gum, guar gum, potassium alginate, sodium alginate, an alginate ester, and pullulan.
<20> The capsule according to any one of <1> to <19>, wherein the capsule film comprises at least one selected from the group consisting of glycerin, sorbitol, erythritol, propylene glycol, polyethylene glycol, and water.
<21> The capsule according to any one of <1> to <20>, wherein the capsule of the present invention is a hard capsule or a soft capsule.
<22> The capsule according to any one of <1> to <21>, wherein the content of the non-animal-derived component in the capsule film is preferably 60 mass% or more, and is preferably 92.5 mass% or less.
<23> The capsule according to any one of <1> to <21>, wherein the capsule is a hard capsule, and the content of the non-animal-derived component in the capsule film is preferably 86 mass% or more, and is preferably 92.5 mass% or less.
<24> The capsule according to any one of <1> to <21>, wherein the capsule is a soft capsule, and the content of the non-animal-derived component in the capsule film is preferably 60 mass% or more, more preferably 62 mass% or more, even more preferably 63 mass% or more, still even more preferably 65 mass% or more, and is preferably 85 mass% or less, more preferably 80 mass% or less, even more preferably 78 mass% or less, still even more preferably 75 mass% or less.
<25> The capsule according to any one of <1> to <24>, wherein the mass ratio of the capsule film to capsule contents, [(capsule film)/(capsule contents)], is preferably from 0.01 to 99, more preferably from 0.01 to 90, more preferably from 0.01 to 80, more preferably from 0.02 to 60, more preferably from 0.02 to 40, more preferably from 0.02 to 20, more preferably from 0.02 to 10, more preferably from 0.05 to 10, more preferably from 0.05 to 5, even more preferably from 0.1 to 5.
<26> The capsule according to any one of <1> to <25>, wherein the ratio of the content of the diacylglycerols in the fat or oil to the content of the non-animal-derived component in the capsule film, [(DAG)/(non-animal-derived component)], is preferably 0.309 or more, and is preferably 1.47 or less.
<27> The capsule according to any one of <1> to <25>, wherein the capsule is a hard capsule, and the ratio of the content of the diacylglycerols in the fat or oil to the content of the non-animal-derived component in the capsule film, [(DAG)/(non-animal-derived component)], is preferably 0.309 or more, more preferably 0.588 or more, and is preferably 0.988 or less, more preferably 0.919 or less, even more preferably 0.876 or less.
<28> The capsule according to any one of <1> to <25>, wherein the capsule is a soft capsule, and the ratio of the content of the diacylglycerols in the fat or oil to the content of the non-animal-derived component in the capsule film, [(DAG)/(non-animal-derived component)], is preferably 0.410 or more, more preferably 0.778 or more, and is preferably 1.47 or less, more preferably 1.31 or less.
<29> The capsule according to any one of <1> to <28>, wherein the ratio of the content of the ω3 unsaturated fatty acids in the constituent fatty acids of the diacylglycerols to the content of the non-animal-derived component in the capsule film, [(ω3)/(non-animal-derived component)], is preferably 0.565 or more, and is preferably 1.05 or less.
<30> The capsule according to any one of <1> to <28>, wherein the capsule is a hard capsule, and the ratio of the content of the ω3 unsaturated fatty acids in the constituent fatty acids of the diacylglycerols to the content of the non-animal-derived component in the capsule film, [(ω3)/(non-animal-derived component)], is preferably 0.565 or more, more preferably 0.593 or more, and is preferably 0.651 or less, more preferably 0.608 or less, even more preferably 0.602 or less.
<31> The capsule according to any one of <1> to <28>, wherein the capsule is a soft capsule, and the ratio of the content of the ω3 unsaturated fatty acids in the constituent fatty acids of the diacylglycerols to the content of the non-animal-derived component in the capsule film, [(ω3)/(non-animal-derived component)], is preferably 0.784 or more, more preferably 0.797 or more, and is preferably 1.05 or less, more preferably 0.805 or less.
<32> The capsule according to any one of <1> to <31>, wherein the ratio of the content of triacylglycerols in the fat or oil to the content of the non-animal-derived component in the capsule film, [(TAG)/(non-animal-derived component)], is preferably 0.0640 or more, and is preferably 1.12 or less.
<33> The capsule according to any one of <1> to <31>, wherein the capsule is a hard capsule, and the ratio of the content of triacylglycerols in the fat or oil to the content of the non-animal-derived component in the capsule film, [(TAG)/(non-animal-derived component)], is preferably 0.144 or more, more preferably 0.155 or more, even more preferably 0.269 or more, and is preferably 0.845 or less, more preferably 0.567 or less.
<34> The capsule according to any one of <1> to <31>, wherein the capsule is a soft capsule, and the ratio of the content of triacylglycerols in the fat or oil to the content of the non-animal-derived component in the capsule film, [(TAG)/(non-animal-derived component)], is preferably 0.0640 or more, more preferably 0.205 or more, and is preferably 1.12 or less, more preferably 0.751 or less.
<35> A capsule, comprising a capsule film including a non-animal-derived component, the capsule film being filled with a fat or oil containing 15 mass% or more of diacylglycerols in which 20 mass% or more of constituent fatty acids are ω3 unsaturated fatty acids.
<36> The capsule according to <35>, wherein the content of an animal-derived protein in components for forming the capsule film other than water is less than 5 mass%, preferably less than 3 mass%, more preferably less than 1 mass%, even more preferably less than 0.1 mass%, and the capsule is still even more preferably substantially free of the anima-derived protein.
<37> The capsule according to <36>, wherein the animal-derived protein is preferably collagen.
<38> A method of suppressing discoloration of a fat or oil, the method comprising filling a capsule film based on a non-animal-derived component or a capsule film including a non-animal-derived component with a fat or oil containing 15 mass% or more of diacylglycerols in which 20 mass% or more of constituent fatty acids are ω3 unsaturated fatty acids.

### Examples

### [Analysis Method]

### (i) Composition of Glycerides in Fat or oil (Fats or oils "a" to "g")

About 10 mg of a fat or oil sample and 0.5 mL of a trimethylsilylating agent ("Silylating agent TH," manufactured by Kanto Chemical Co., Inc.) were loaded into a sample bottle made of glass, and the bottle was sealed and heated at 70°C for 15 minutes. 1.0 mL of water and 1.5 mL of hexane were added thereto, and the bottle was shaken. The bottle was left at rest, and then the upper layer was analyzed by gas-liquid chromatography (GLC).

### <GLC Analysis Conditions>

### (Conditions)

Apparatus: Agilent 7890B (manufactured by Agilent Technologies)
Column: DB-1ht 10 m×0.25 mm×0.2 µm (manufactured by Agilent J&W)
Carrier gas: 1.0 mL He/min
Injector: Split (1:50), T=340°C
Detector: FID, T=350°C
Oven temperature: increased from 80°C at 10°C/min to 340°C, and kept for 15 min

### (ii) Composition of Glycerides in Fat or oil (Fat or oil "h")

A fat or oil was dissolved at 1 mg/ml with a chloroform/methanol (1/9) mixed solvent, and the solution was diluted 10-fold with methanol, followed by analysis through high-performance liquid chromatography (HPLC) together with standard samples for correction.

### <Standard Samples>

(1) For diacylglycerols: triolein/diolein/monoolein=5/80/15 (weight ratio)
(2) For triacylglycerols: triolein/diolein/monoolein=90/5/5 (weight ratio)

### <HPLC Analysis Conditions>

### (Conditions)

Apparatus: UHPLC (manufactured by Thermo Fisher Scientific Inc.)
Column: L-column C8 (2.1 mm×35 mm×5 µm) (manufactured by
Chemicals Evaluation and Research Institute, Japan)
Column temperature: 40°C
Sample injection volume: 10 µL
Flow rate: 0.5 mL/min
Mobile phase: (A) water/acetonitrile=9/1; (B) isopropanol

**Table 1**

| (Gradient Table) | | |
|---|---|---|
| min | A [%] | B [%] |
| 0 | 100 | 0 |
| 1 | 50 | 50 |
| 8 | 0 | 100 |
| 10 | 0 | 100 |
| 10.1 | 100 | 0 |
| 15 | 100 | 0 |

### Detector: charged aerosol detector (CAD)

### (iii) Composition of Constituent Fatty Acids of Fat or oil

Fatty acid methyl esters were prepared in accordance with "Preparation method for fatty acid methyl ester (2.4.1.-1996)" described in "Standard Method for the Analysis of Fats, Oils and Related Materials" edited by Japan Oil Chemists' Society, and the resultant fat or oil samples were subjected to measurement in conformity with American Oil Chemists. Society Official Method Ce 1f-96 (GLC method).

### <GLC Analysis Conditions>

Apparatus: Agilent 7890B (manufactured by Agilent Technologies)
Column: CP-SIL88 50 m×0.25 mm×0.2 µm ((manufactured by Agilent J&W)
Carrier gas: 1.0 mL He/min
Injector: Split (1:50), T=300°C
Detector: FID, T=300°C
Oven temperature: kept at 150°C for 5 min→increased at 1°C/min→kept at 160°C for 5 min→increased at 2°C/min→kept at 200°C for 10 min→increased at 10°C/min→kept at 220°C for 5 min

### [Preparation of Fats or oils]

Fats or oils "a" to "h" shown in Table 2 were prepared.

**Table 2**

| Fat or oil | | Fat or oil "a" | Fat or oil "b" | Fat or oil "c" | Fat or oil "d" | Fat or oil "e" | Fat or oil "f" | Fat or oil "g" | Fat or oil "h" |
|---|---|---|---|---|---|---|---|---|---|
| Composition of glycerides (%) | Free fatty acids | 0.1 | 0.2 | 0.2 | 0.2 | 0.2 | 0.2 | 0.3 | 0.2 |
| | MAG | 0.8 | 1.5 | 0.6 | 0.4 | 1.5 | 1.5 | 0.2 | 1.4 |
| | DAG | 2.7 | 85.0 | 26.6 | 50.6 | 85.0 | 85.0 | 95.4 | 75.3 |
| | TAG | 96.4 | 13.3 | 72.7 | 48.8 | 13.3 | 13.3 | 4.2 | 23.1 |
| Fatty acid composition of fat or oil (%) | Palmitic acid | 5.4 | 2.5 | 4.6 | 3.9 | 2.5 | 2.5 | 2.1 | 17.9 |
| | Stearic acid | 4.5 | 1.5 | 3.7 | 3.0 | 1.5 | 1.5 | 0.9 | 1.7 |
| | Oleic acid | 18.7 | 25 | 21.4 | 23.0 | 25 | 25 | 12.1 | 10 |
| | Linoleic acid | 14.4 | 16.1 | 14.9 | 15.5 | 16.1 | 16.1 | 13.5 | 1.1 |
| | ALA | 54.7 | 49.7 | 53.5 | 52.3 | 49.7 | 49.7 | 67.9 | 0.0 |
| | DHA | 0.0 | 0.0 | 0.0 | 0.0 | 0.0 | 0.0 | 0.0 | 17.5 |
| | EPA | 0.0 | 0.0 | 0.0 | 0.0 | 0.0 | 0.0 | 0.0 | 33.4 |
| | Others | 2.3 | 5.2 | 2.0 | 2.4 | 5.2 | 5.2 | 3.5 | 18.4 |
| Fatty acid composition in DAG (%) | Palmitic acid | 6.0 | 2.4 | 2.8 | 2.5 | 2.4 | 2.4 | 1.9 | 18.4 |
| | Stearic acid | 3.5 | 1.3 | 1.6 | 1.3 | 1.3 | 1.3 | 0.8 | 1.6 |
| | Oleic acid | 25.9 | 24 | 25.4 | 25.7 | 24 | 24 | 11.4 | 9.5 |
| | Linoleic acid | 22.8 | 16.2 | 16.9 | 16.2 | 16.2 | 16.2 | 13.7 | 0.8 |
| | ALA | 38.8 | 52.3 | 51.0 | 51.8 | 52.3 | 52.3 | 68.1 | 0.0 |
| | DHA | 0.0 | 0.0 | 0.0 | 0.0 | 0.0 | 0.0 | 0.0 | 19.3 |
| | EPA | 0.0 | 0.0 | 0.0 | 0.0 | 0.0 | 0.0 | 0.0 | 36.7 |
| | Others | 3.0 | 3.8 | 2.3 | 2.5 | 3.8 | 3.8 | 4.1 | 13.7 |
| Antioxidant (%) | Tocopherol | - | - | - | - | 0.01 | 0.1 | 0.2 | 0.2 |
| | Ascorbyl palmitate | - | - | - | - | 0.01 | 0.1 | 0.1 | 0.1 |
| | Rosemary extract | - | - | - | - | - | - | 0.3 | 0.3 |

| | | | | | | | | | |
|---|---|---|---|---|---|---|---|---|---|
| MAG: Monoacylglycerols DAG: Diacylglycerols TAG: Triacylglycerols | | | | | | | | | |

Fat or oil "a": Refined linseed oil manufactured by The Nisshin OilliO Ltd. was used.

Fat or oil "b": Linseed oil manufactured by Archer-Daniels-Midland Company was hydrolyzed with an enzyme to provide fatty acids, followed by stepwise cooling, and the precipitated fatty acids were fractionated by centrifugation. Next, an esterification reaction between the fractionated fatty acids and glycerin was performed under reduced pressure through use of an immobilized 1,3-selective lipase as a catalyst. After the immobilized enzyme had been separated by filtration, the product after the completion of the reaction was subjected to molecular distillation, water washing, and deodorization to provide a fat or oil "b".

Fat or oil "c": The fat or oil "a" and the fat or oil "b" were mixed at a ratio of 70:30 to provide a fat or oil "c".

Fat or oil "d": The fat or oil "a" and the fat or oil "b" were mixed at a ratio of 40:60 to provide a fat or oil "d".

Fat or oil "e": 0.01% of tocopherol (manufactured by Riken Vitamin Co., Ltd.) and 0.01% of ascorbyl palmitate (manufactured by DSM) were added to the fat or oil "b" to provide a fat or oil "e".

Fat or oil "f": 0.1% of tocopherol and 0.1% of ascorbyl palmitate were added to the fat or oil "b" to provide a fat or oil "f".

Fat or oil "g": A fat or oil was produced by the same method as that of the fat or oil "b" through use of high linolenic acid linseed oil manufactured by Summit Oil Mill Co., Ltd., and then 0.2% of tocopherol, 0.1% of ascorbyl palmitate, and 0.3% of rosemary extract (manufactured by Givaudan) were added to provide a fat or oil "g".

Fat or oil "h": 80 Parts by mass of algae oil manufactured by DSM and 25 parts by mass of glycerin were mixed, and a glycerolysis reaction was performed by using calcium hydroxide as a catalyst. After that, molecular distillation, acid treatment, and water washing were performed to provide a treated oil. The treated oil was deodorized, and then 0.2% of tocopherol, 0.1% of ascorbyl palmitate, and 0.3% of rosemary extract were added to provide a fat or oil "h".

### Examples 1 to 5 and Comparative Examples 1 to 3

### [Preparation of Capsules]

Capsule films each having the composition shown in Table 3 were purchased and used for evaluations. The capsule film (No. 1) was filled with 350 mg of each of the fats or oils prepared above to provide a capsule. The resulting capsule was evaluated with reference to the color. Each of the capsules of Examples 1 to 5 and Comparative Examples 1 and 2 was evaluated with reference to the oxidative stability. The results are shown in Table 4.

**Table 3**

| | | Film "a" | Film "b" | Film "c" |
|---|---|---|---|---|
| Product name | | Corn capsule | MP capsule | HF capsule |
| Manufacturer | | Matsuya | Matsuya | Matsuya |
| | Pullulan | 85.5 | 0 | 0 |
| Raw materials (%) | HPMC | 0 | 91.2 | 0 |
| | Carrageenan | 0.5 | 1.3 | 0 |
| | Pork gelatin | 0 | 0 | 100 |
| | Potassium chloride | 2 | 1 | 0 |
| | Others | 12 | 6.5 | 0 |

### [Color Evaluation]

### (i) Sensory Evaluation

20 Capsules of each of Examples and Comparative Examples were stored in 10 mL glass vials, with 5 capsules per vial, at 60°C for 3 days. Then, four expert panelists visually evaluated the colors of the capsules in accordance with the following criteria. A score was determined by consultation among the expert panelists.

### Color Evaluation Criteria

Score 1: Almost no change from the initial state was observed.
Score 2: A slight color change from the initial state was observed.
Score 3: A color change from the initial state was observed.
Score 4: A significant color change from the initial state was observed.

### (ii) Measurement of Gardner Color Number

20 Capsules of each of Examples and Comparative Examples were stored in 10 mL glass vials, with 5 capsules per vial, at 60°C for 3 days. Then, the color of the fat or oil was measured with a color difference meter (CR-5). The difference between the color tones of the fat or oil immediately after the preparation of the capsule and after the storage thereof was determined as the amount of change. The average of the 20 capsules was determined.

### [Oxidative Stability Evaluation]

20 Capsules of each of Examples and Comparative Examples were stored in 10 mL glass vials, with 5 capsules per vial, at 60°C for 3 days. Then, the peroxide value (POV) of the fat or oil was measured in accordance with Standard Methods for Analysis of Fats, Oils and Related Materials 2.5.2.1. The difference between the peroxide values (POVs) of the fat or oil immediately after the preparation of the capsule and after the storage thereof was determined as the amount of change. The average of the 20 capsules was determined.

**Table 4**

| | | Example 1 | Example 2 | Example 3 | Example 4 | Example 5 | Comparative Example 1 | Comparative Example 2 | Comparative Example 3 |
|---|---|---|---|---|---|---|---|---|---|
| Capsule film | | Film "a" | Film "b" | Film "a" | Film "a" | Film "a" | Film "a" | Film "b" | Film "c" |
| Animal-derived /non-animal-derived | | Non-animal-derived | Non-animal-derived | Non-animal-derived | Non-animal-derived | Non-animal-derived | Non-animal-derived | Non-animal-derived | Animal-derived |
| Fat or oil | | Fat or oil "b" | Fat or oil "b" | Fat or oil "c" | Fat or oil "d" | Fat or oil "e" | Fat or oil "a" | Fat or oil "a" | Fat or oil "b" |
| DAG in fat or oil [%] | | 85.0 | 85.0 | 26.6 | 50.6 | 85.0 | 2.7 | 2.7 | 85.0 |
| ALA in DAG [%] | | 52.3 | 52.3 | 51.0 | 51.8 | 52.3 | 38.8 | 38.8 | 52.3 |
| Sensory evaluation | Color | 1 | 2 | 1 | 1 | 1 | 2 | 3 | 4 |
| Gardner (amount of change) | | 0.1 | 0.2 | 0.1 | 0.2 | 0 | 0.2 | 0.4 | 1.6 |
| POV (amount of change) | | 4.9 | 15.7 | 13.8 | 9.5 | 2.5 | 20.4 | 28.8 | - |

### Example 6 and Comparative Example 4

### [Preparation of Capsules]

A capsule film (No. 1) having the composition shown in Table 3 was filled with 350 mg of each of the fats or oils prepared above to provide a capsule. The resulting capsule was evaluated with reference to the color. The capsule prepared in Example 6 was evaluated with reference to the oxidative stability.

### [Evaluations of Color and Oxidative Stability]

The capsules were stored in 10 mL glass vials, with 5 capsules per vial, at 60°C for 2 days. Then, the color of each of the capsules was evaluated by the same procedure as that of Example 1. The peroxide value of the fat or oil was measured by the method described above. The results are shown in Table 5.

**Table 5**

| | | Example 6 | Comparative Example 4 |
|---|---|---|---|
| Capsule film | | Film "a" | Film "c" |
| Animal-derived/non-animal-derived | | Non-animal-derived | Animal-derived |
| Fat or oil | | Fat or oil "h" | Fat or oil "h" |
| DAG in fat or oil [%] | | 75.3 | 75.3 |
| EPA and DHA in DAG [%] | | 56.0 | 56.0 |
| Sensory evaluation | Color | 1 | 4 |
| Gardner (amount of change) | | 0.2 | 1.2 |
| POV (amount of change) | | 5.3 | - |

### Examples 7 to 15 and Comparative Examples 5 to 7

Model films "a" to "e" shown in Table 6 were prepared.

**Table 6**

| | | Model film "a" | Model film "b" | Model film "c" | Model film "d" | Model film "e" |
|---|---|---|---|---|---|---|
| Film raw materials (%) | Pork gelatin | 70 | 0 | 0 | 0 | 0 |
| | Dextrin A | 0 | 50 | 0 | 0 | 0 |
| | Dextrin B | 0 | 0 | 50 | 0 | 0 |
| | Modified starch A | 0 | 0 | 0 | 50 | 0 |
| | Modified starch B | 0 | 0 | 0 | 0 | 50 |
| | Carrageenan | 0 | 15 | 15 | 15 | 15 |
| | Glycerin | 30 | 35 | 35 | 35 | 35 |
| Purified water | | q.s. | q.s. | q.s. | q.s. | q.s. |

| | | | | | | |
|---|---|---|---|---|---|---|
| Pork gelatin: Tomizawa Shouten, Gelatin Powder (pork gelatin) Dextrin A: Sandec #30, manufactured by Sanwa Starch Co., Ltd. Dextrin B: Cluster Dextrin, manufactured by Glico Nutrition Co., Ltd. Modified starch A: Starch sodium octenyl succinate (Emulstar A1), manufactured by Matsutani Chemical Industry Co., Ltd. Modified starch B: Hydroxypropylated phosphate-crosslinked starch (BINASOL 70C), manufactured by Tokai Starch Co., Ltd. Carrageenan: GENUTINE 310-C, manufactured by Sansho Co., Ltd. Glycerin: GLYCERIN (FOOD ADDITIVE), manufactured by Kao Corporation | | | | | | |

Model film "a": After the raw materials for film were mixed at the ratio shown in Table 6, purified water was added thereto, and the mixture was heated at 70°C for 10 minutes. After heat dissolution, the resultant was dried in a dryer (AG-WDN) to prepare a model film "a" for a capsule.

Model films "b" to "e": After the raw materials for film were mixed at the ratio shown in Table 6, purified water was added thereto, and the mixture was heated at 80°C for 10 minutes. After heat dissolution, the resultant was dried in a dryer (AG-WDN) to prepare a model film for a capsule.

The following color evaluation was performed. In addition, the oxidative stability of each of the model films of Examples 7 to 15 and Comparative Examples 5 and 6 was evaluated. The results are shown in Table 7.

### [Color Evaluation]

### (i) Sensory Evaluation

0.5 g of the model film and 10 g of the fat or oil were loaded into a 50 mL glass vial and stored at 60°C for 2 days. Then, four expert panelists visually evaluated the color of the fat or oil in accordance with the following criteria. A score was determined by consultation among the expert panelists.

### Color Evaluation Criteria

Score 1: Almost no change from the initial state was observed.
Score 2: A slight color change from the initial state was observed.
Score 3: A color change from the initial state was observed.
Score 4: A significant color change from the initial state was observed.

### (ii) Measurement of Gardner Color Number

0.5 g of the model film was immersed in 10 g of the fat or oil in a 50 mL glass vial, and the resultant was stored at 60°C for 2 days. Then, the color of the fat or oil was measured with a color difference meter (CR-5). The difference between the color tones of the fat or oil before and after the storage was determined as the amount of change.

### [Oxidative Stability Evaluation]

0.5 g of the model film was immersed in 10 g of the fat or oil in a 50 mL glass vial, and the resultant was stored at 60°C for 2 days. Then, the peroxide value (POV) of the fat or oil was measured in accordance with Standard Methods for Analysis of Fats, Oils and Related Materials 2.5.2.1. The difference between the peroxide values (POVs) of the fat or oil before and after the storage was determined as the amount of change.

**Table 7**

| | | Example 7 | Example 8 | Example 9 | Example 10 | Example 11 | Example 12 | Example 13 | Example 14 | Example 15 | Comparative Example 5 | Comparative Example 6 | Comparative Example 7 |
|---|---|---|---|---|---|---|---|---|---|---|---|---|---|
| Model film | | Film "b" | Film "c" | Film "d" | Film "e" | Film "b" | Film "b" | Film "b" | Film "b" | Film "b" | Film "b" | Film "e" | Film "a" |
| Animal-derived/non-animal-derived | | Non-animal-derived | Non-animal-derived | Non-animal-derived | Non-animal-derived | Non-animal-derived | Non-animal-derived | Non-animal-derived | Non-animal-derived | Non-animal-derived | Non-animal-derived | Non-animal-derived | Animal-derived |
| Fat or oil | | Fat or oil "b" | Fat or oil "b" | Fat or oil "b" | Fat or oil "b" | Fat or oil "c" | Fat or oil "d" | Fat or oil "e" | Fat or oil "f" | Fat or oil "g" | Fat or oil "a" | Fat or oil "a" | Fat or oil "b" |
| DAG in fat or oil [%] | | 85.0 | 85.0 | 85.0 | 85.0 | 26.6 | 50.6 | 85.0 | 85.0 | 95.4 | 2.7 | 2.7 | 85.0 |
| ALA in DAG [%] | | 52.3 | 52.3 | 52.3 | 52.3 | 51.0 | 51.8 | 52.3 | 52.3 | 68.1 | 38.8 | 38.8 | 52.3 |
| Sensory evaluation | Col or | 2 | 2 | 2 | 2 | 2 | 2 | 1 | 1 | 2 | 2 | 2 | 4 |
| Gardner (amount of change) | | 0.4 | 0.4 | 0.5 | 0.5 | 0.6 | 0.6 | 0.1 | 0.1 | 0.7 | 0.9 | 1.0 | 1.4 |
| POV (amount of change) | | 19.8 | 19.4 | 20.7 | 20.3 | 28.6 | 27.2 | 0.5 | 0.0 | 0.0 | 46.5 | 46.7 | - |

As is apparent from the results shown in Table 4, Table 5, and Table 7, it was found that the capsule containing the fat or oil of the present invention in a capsule film based on a non-animal-derived component had less discoloration of the fat or oil, as compared to a capsule using gelatin as a film base. In addition, the oxidative stability of the fat or oil of the present invention was excellent.

### Formulation Examples 1 to 14

Soft capsules were prepared by filling a soft capsule film having the composition shown in Table 8 with 100 mg to 1 500 mg of each of the fats or oils "b" to "h" prepared above in accordance with a conventional method.

**Table 8**

| (mass%) | Formulation Example | | | | | | | | | | | | | |
|---|---|---|---|---|---|---|---|---|---|---|---|---|---|---|
| | 1 | 2 | 3 | 4 | 5 | 6 | 7 | 8 | 9 | 10 | 11 | 12 | 13 | 14 |
| Acid-hydrolyzed starch*¹ | 40 | 47 | 48 | 47 | 50 | 54 | 55 | 40 | 47 | 48 | 47 | 50 | 54 | 55 |
| 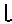 -Carrageenan | 25 | 15 | 15 | 10 | 17 | 20 | 20 | | | | 5 | | | |
| x-Carrageenan | | | | 5 | | | | 25 | 15 | 15 | 10 | 17 | 20 | 20 |
| Glycerin | 35 | 37 | 37 | 37 | 33 | 25 | 25 | 35 | 37 | 37 | 37 | 33 | 25 | 25 |
| Sodium phosphate | | 1 | | 1 | | 1 | | | 1 | | 1 | | 1 | |
| Total | 100.0 | 100.0 | 100.0 | 100.0 | 100.0 | 100.0 | 100.0 | 100.0 | 100.0 | 100.0 | 100.0 | 100.0 | 100.0 | 100.0 |

| | | | | | | | | | | | | | | |
|---|---|---|---|---|---|---|---|---|---|---|---|---|---|---|
| *1: WS-10 | | | | | | | | | | | | | | |

### Formulation Examples 15 to 20

Soft capsules were prepared by filling a soft capsule film having the composition shown in Table 9 with 100 mg to 1 500 mg of each of the fats or oils "b" to "h" prepared above in accordance with a conventional method.

**Table 9**

| (mass%) | Formulation Example | | | | | |
|---|---|---|---|---|---|---|
| | 15 | 16 | 17 | 18 | 19 | 20 |
| Acid-hydrolyzed starch^{*1} | 40 | 47 | 48 | 50 | 54 | 55 |
| λ-Carrageenan | 25 | 15 | 15 | 17 | 20 | 20 |
| Glycerin | 35 | 37 | 37 | 33 | 25 | 25 |
| Sodium phosphate | | 1 | | | 1 | |
| Total | 100.0 | 100.0 | 100.0 | 100.0 | 100.0 | 100.0 |

| | | | | | | |
|---|---|---|---|---|---|---|
| *1: WS-10 | | | | | | |

### Formulation Examples 21 to 40

Soft capsules were prepared by filling a soft capsule film having the composition shown in Table 10 with 100 mg to 1 500 mg of each of the fats or oils "b" to "h" prepared above in accordance with a conventional method.

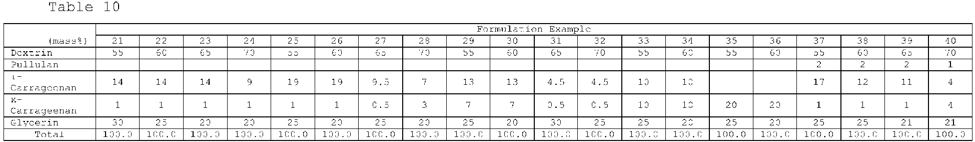

### Formulation Examples 41 to 45

Soft capsules were prepared by filling a soft capsule film having the composition shown in Table 11 with 100 mg to 1 500 mg of each of the fats or oils "b" to "h" prepared above in accordance with a conventional method.

**Table 11**

| (mass%) | Formulation Example | | | | |
|---|---|---|---|---|---|
| | 41 | 42 | 43 | 44 | 45 |
| Phosphate-crosslinked hydroxypropylated starch | | 70 | | 70 | 35 |
| Acid-modified hydroxypropylated starch | 70 | | 70 | | 30 |
| ι-Carrageenan | 9 | 9 | 5 | 5 | 2.5 |
| κ-Carrageenan | 1 | 1 | 0 | 0 | 2.5 |
| Glycerin | 20 | 20 | 25 | 25 | 30 |
| Total | 100.0 | 100.0 | 100.0 | 100.0 | 100.0 |

### Formulation Examples 46 to 65

Soft capsules were prepared by filling a soft capsule film having the composition shown in Table 12 with 100 mg to 1 500 mg of each of the fats or oils "b" to "h" prepared above in accordance with a conventional method.

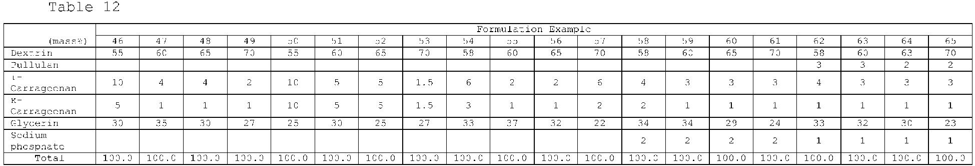

### Formulation Examples 66 to 75

Soft capsules were prepared by filling a soft capsule film having the composition shown in Table 13 with 100 mg to 1 500 mg of each of the fats or oils "b" to "h" prepared above in accordance with a conventional method.

**Table 13**

| (mass%) | Formulation Example | | | | | | | | | |
|---|---|---|---|---|---|---|---|---|---|---|
| | 66 | 67 | 68 | 69 | 70 | 71 | 72 | 73 | 74 | 75 |
| Dextrin | 45 | 32 | 10 | | | 40 | 20 | | | |
| Galactan | 30 | 35 | 50 | 60 | 70 | 30 | 40 | 50 | 60 | 70 |
| Guar gum | 10 | 10 | 15 | | | 15 | 20 | 25 | 20 | 9 |
| Glycerin | 15 | 23 | 25 | 40 | 30 | 15 | 20 | 25 | 20 | 21 |
| Total | 100.0 | 100.0 | 100.0 | 100.0 | 100.0 | 100.0 | 100.0 | 100.0 | 100.0 | 100.0 |

### Formulation Examples 76 to 81

Soft capsules were prepared by filling a soft capsule film having the composition shown in Table 14 with 100 mg to 1 500 mg of each of the fats or oils "b" to "h" prepared above in accordance with a conventional method.

**Table 14**

| (mass%) | Formulation Example | | | | | |
|---|---|---|---|---|---|---|
| | 76 | 77 | 78 | 79 | 80 | 81 |
| Dextrin | 45 | 50 | 52 | 45 | 50 | 52 |
| ι-Carrageenan | 13 | 15 | 18 | 13 | 15 | 18 |
| Gum arabic | 2 | 2 | 2 | 1.9 | 1.9 | 1.9 |
| Alginate ester | | | | 0.1 | 0.1 | 0.1 |
| Glycerin | 40 | 33 | 28 | 40 | 33 | 28 |
| Total | 100.0 | 100.0 | 100.0 | 100.0 | 100.0 | 100.0 |

### Formulation Examples 82 to 86

Soft capsules were prepared by filling a soft capsule film having the composition shown in Table 15 with 100 mg to 1 500 mg of each of the fats or oils "b" to "h" prepared above in accordance with a conventional method.

**Table 15**

| (mass%) | Formulation Example | | | | |
|---|---|---|---|---|---|
| | 82 | 83 | 84 | 85 | 86 |
| Indigestible dextrin | 35 | 35 | 40 | 40 | 40 |
| Phosphate-crosslinked hydroxypropylated starch | 30 | | | 10 | |
| Wet-heat-treated starch | | 30 | 30 | 10 | 20 |
| Gellan gum | | | | | 15 |
| Locust bean gum | 7.5 | 5 | 5 | 7.5 | |
| Xanthan gum | 7.5 | 5 | 5 | 7.5 | |
| Glycerin | 20 | 25 | 20 | 25 | 25 |
| Total | 100.0 | 100.0 | 100.0 | 100.0 | 100.0 |

## Claims

1. A capsule, comprising a capsule film based on a non-animal-derived component, the capsule film being filled with a fat or oil containing 15 mass% or more of diacylglycerols in which 20 mass% or more of constituent fatty acids are ω3 unsaturated fatty acids.

2. The capsule according to claim 1, wherein the non-animal-derived component comprises at least one selected from the group consisting of a plant-derived component, a seaweed-derived component, and a microorganism-derived component.

3. The capsule according to claim 1, wherein the non-animal-derived component comprises at least one selected from the group consisting of starch, modified starch, dextrin, gum arabic, pectin, a cellulose derivative, glucomannan, carrageenan, gellan gum, xanthan gum, locust bean gum, guar gum, potassium alginate, sodium alginate, an alginate ester, and pullulan.

4. The capsule according to any one of claims 1 to 3, wherein the ω3 unsaturated fatty acid is at least one selected from the group consisting of α-linolenic acid, eicosapentaenoic acid, and docosahexaenoic acid.
